# EUROPEAN PATENT APPLICATION

(11) **EP 1 273 308 A1**
(43) Date of publication of application: **08.01.2003**
(21) Application number: 01202549.0
(22) Date of filing: 03.07.2001
(51) Int. Cl.: A61K 47/48, A61K 7/42

(54) **Peptide-flavonoid conjugates, their preparation and use in uv protection**

(71) Applicant: Fuji Photo Film B.V., 5000 LJ Tilburg (NL)
(72) Inventor: Bouwstra, Jan Bastiaan, NL-3721 AJ Bilthoven (NL); Toda, Yuzo, NL-5051 KZ Goirle (NL)
(74) Representative: Jorritsma, Ruurd

(57) **Abstract**

The invention pertains to a UV radiation absorbing compound wherein at least one flavonoid is covalently linked to a polypeptide. The link can be constituted by an alkylidene group attached to a nitrogen atom of the polypeptide and to an aromatic carbon atom of the flavonoid molecule. It can be produced by a Mannich type of reaction involving a primary or secondary amine and flavonoid compound such as quercetin.

## Description

### FIELD OF THE INVENTION

This invention relates to a novel light-stable polypeptide for protection against UV-radiation and its detrimental effects. It further relates to the synthesis and cosmetic use of said light-stable polypeptide.

### BACKGROUND

The detrimental effects of exposing the skin to UV light are manifold and are well documented in the prior art. It has been long recognized that UV-B radiation, with a wavelength of 290 to 320 nm, causes erythema or sunburn. It was not until around 1980 that it was discovered that UV-A radiation, with a wavelength from 320 to 400 nm, causes phototoxic and photochemical reactions.

While about 70% UV-B radiation is blocked by the outer skin or stratum corneum, this is not the case for UV-A radiation, which can subsequently penetrate deep into the living dermis. A well known destructive effect of UV-A is oxidative stress. Superoxide, which is formed by UV-A radiation, can release iron from ferritin, an iron-storage protein located in fibroblasts in the skin (Pourzand et al, Proc. Natl. Acad. Sci. USA, June 1999, Vol 96, p. 6751-56). The role of iron in the Fenton or Haber Weis reaction resulting in the production of highly destructive hydroxyl radicals and hydrogen peroxide is well known. Also other metal ions like copper-ions have been reported to catalyze the formation of oxygen radicals. The role of these destructive products in damaging DNA is well known as for example described by Sestili et al (Free Radical Biology & Medicine [US], July 15 1998, 25, [2] p.196-200).

The Fenton or Haber Weis reaction can be effectively prevented by chelating iron. Certain flavonoid compounds, like quercetin, are known metal chelators and radical scavengers. They have been effectively employed to reduce oxidative stress, thereby preventing or limiting melanoma or non-melanoma carcinomas (Caltagirone et al, Int. J. of Cancer, 2001, V87, N4 [Aug 15], p. 595-600). The advantage of quercetin over other metal chelators is that it is a better scavenger, and has no skin-irritating side effects. Furthermore, it can also function as a radical scavenger, preventing the release of iron from ferritin by superoxide radicals.

The normal biochemical protection by enzymes like superoxide dismutase is not sufficient to effectively stop the reaction induced by UV-A radiation. Hence the necessity to protect the skin from these harmful effects is still mandatory. Other oxidative stress phenomena which are prevented or reduced by quercetin are damaging of collagen resulting for example in accelerated skin aging and white spots, or damaging of cell walls by lipid peroxidation.

The metal chelating property of quercetin was found to be related to the presence of a catechol group and the presence of a 4-keto, 5-hydroxy region (Chenh, Breen, Biometals (Netherlands) March 2000, 13 [1] p. 77-83). Aherne and O'Brien (Free radical Biology and Medicine, 2000, V29, N6 [Sep 15], p507-514) report that rutin, in which the 3-hydroxy group of quercetin is replaced by a diglycoside group, does not chelate metal ions as effectively as quercetin and does not have the radical scavenging capability of quercetin.

A disadvantage of flavones and flavanones is their low water solubility. Therefore, they are unsuitable for use in water-based cosmetics, like hydrogels. They require the use of organic solvents, which generally exhibit unwanted side effects. A further disadvantage of UV absorbing compounds in general is that they are themselves unstable under UV light. UV exposure can cause photochemical reactions that destroy the UV-absorbing compound thereby reducing the protection against UV radiation. A further disadvantage of flavonoids described in the prior art is that these compounds may have the risk of penetrating through the skin to enter the bloodstream.

The use of quercetin or its derivatives as a UV-A filter is described in the prior art. US 5,565,435 describes an alpha-glycosyl quercetin. A better UV stability and water solubility is claimed. However, such a compound is expected to have reduced capability as a metal chelator and may not function as a radical scavenger (Aherne and O'Brien (Free radical Biology and Medicine, 2000, V29, N6 [Sep 15], p507-514).

US 5,718,906 discloses a cosmetic composition for protection against UV radiation containing a tetra-alkylated quercetin. Although it is stated that these compounds can prevent cancer, it is unlikely that the alkylated quercetin can chelate metal ions, since both the catechol- and other hydroxy groups have been alkylated.

EP 1,055,415 A2, EP 1,055,411 A2 and EP 1,055,412 A2 disclose the use of flavonoids in combination with a stabilizer, which also prevents breakdown of flavonoids by UV light at high concentrations, preferring structures in which the 3-hydroxy group is glycosylated. As described above, these structures cannot be expected to chelate iron as well as quercetin.

WO 99/25316 discloses a composition comprising a combination of a flavone and a flavanone as a sunscreen filter. The role of these compounds as metal chelators or radical scavengers is not disclosed here.

US 5,403,944 discloses an organopolysiloxane polymer with UV-absorbing groups, the molecular weight of which is such that it cannot penetrate in or through the skin.

In spite of the attempts described above, there still remains a need for UV filters which provide sufficient direct protection from sunlight by blocking the radiation while simultaneously preventing or reducing the detrimental effect of the remaining unblocked radiation.

### SUMMARY

It is an object of this invention to provide a new water-soluble UV-absorbing flavonoid compound.

It is also an objective of this invention to provide a method to synthesize said new water-soluble UV-absorbing flavonoid compound.

A further object of the invention is to provide a new water-soluble UV-absorbing flavonoid compound that can be applied in high concentrations with high stability against breakdown under UV light.

Another object of the invention is to provide a new flavonoid compound providing optimal protection from UV radiation by absorbing said radiation and the damaging effects of said radiation by chelating metal ions and scavenging oxygen radicals.

Another object of the invention is to provide a new flavonoid compound that can be safely applied to the skin with minimal risk of entering the bloodstream.

The present invention is based on the surprising insight that all of the objectives could be reached by a UV radiation absorbing biopolymer comprising at least one flavonoid compound linked to a water-soluble biopolymer. The UV-absorbing biopolymer is capable of chelating metal ions like iron or copper. The biopolymer is preferably a polypeptide or protein such as gelatin.

The new UV absorbing polymer can be applied without significant risk of entering the bloodstream, preventing the risk of immune reactions or other detrimental effects.

### DETAILED DESCRIPTION

The present invention is directed to UV radiation absorbing compounds that can be applied for example in cosmetics to protect the human skin or hair from the detrimental effects of exposure to sunlight. The present invention is also directed to stable UV radiation absorbing compounds that are water-soluble and can be applied in concentrations high enough to provide sufficient protection.

The present invention is further directed to compounds that are capable of protecting the skin or hair from effects like oxidative stress by exhibiting metal ion chelating or radical scavenging properties.

The present invention is especially directed to stable water-soluble UV radiation absorbing compounds that are prevented from penetrating through the skin into the bloodstream.

Preventing said UV absorbing polymer from entering the bloodstream reduces the risk of immune reactions or other detrimental effects.

Although flavonoids like quercetin have been reported to have many beneficial effects, there is some doubt about the possible carcinogenic effects of such compound when injected, as described by Drewa et al (Neoplasma, 2001, V48, N1, p12-18). It is therefore prudent to prevent such compounds from penetrating through the skin and entering the bloodstream. In this respect also the use of organic solvents in cosmetic applications is to be avoided since such solvents, besides eliciting immune reactions like skin hypersensitivity, may increase skin penetration by facilitating transport of harmful substances through the lamellar intercellular lipid barrier of the stratum corneum.

Surprisingly, a new UV radiation absorbing polymer comprising at least one flavonoid compound linked to a water-soluble biopolymer, was found which exhibits all of the desired properties.

In a preferred embodiment the UV radiation absorbing biopolymer is a polypeptide. Within the context of this invention, a polypeptide is to be understood to comprise any molecule having at least 20 amino acids linked by peptide bonds, including natural proteins, denatured proteins, synthetic and recombinant proteins and peptides, glycoproteins, proteoglycans, lipoproteins and other molecules which may contain other groups, including bio-oligomer or biopolymer groups as a side chain or in the chain. The maximum length is usually 2000 amino acids. In a preferred embodiment, the polypeptide contains between 30 and 1000 amino acids. Polypeptides, especially of the gelatin type, having between 50 and 500 amino acids are especially preferred.

In a preferred embodiment, the UV-absorbing compound comprises a flavanol (3-hydroxy-flavone) compound, capable of chelating iron, like quercetin, linked to a polypeptide. A more preferred embodiment comprises a flavanol and a flavanone compound capable of chelating iron ions and respectively absorbing UV-A and UV-B radiation. The ratio flavone/flavanone can be adjusted to determine the ratio UV-B/UV-A protection. A high flavanone load will provide good UV-A protection, but will also stop tanning of the skin.

WO 99/25316 teaches that flavanone/flavone ratios from 10:1 to 25:1 (w/w) provides sufficient protection against UV-A while allowing the skin to tan.

In another embodiment one of said flavanol or flavanone compounds is capable of chelating iron ions. It is also possible to link a suitable conventional UV-absorbing compound as described for example in EP 1,055,415, in addition to the flavonoid compound(s). The possibility to link only conventional UV-absorbing compounds and optionally metal chelating compounds was considered, but dismissed in the light of the simplicity of the solution provided by our invention.

In yet another embodiment the flavonoid molecules are chemically modified to reduce the yellow color which is caused by the presence of the hydroxyl groups. Such a modification can be any of alkylation, hydroxyalkylation, acylation, or the like or a combination of such modifications. Modification can be done on any of the hydroxyl groups as long as the metal chelating function of the catechol groups is not compromised. In this respect there is a preference for rutin, in which the 3-hydroxy group is substituted by a disaccharide. An additional benefit of such a modification is that solubility of the flavonoid compound is increased. Modification can be performed before or after linking said flavonoid to a polypeptide.

The amount of flavonoid that should be linked to the biopolymer is determined by several factors. The load can be between 0.15 and 15 flavonoid molecules per 100 amino acids. At lower loads the amount of polypeptide, in the form of a cream or (hydro)gel or the like, which is put on the skin to achieve the required UV protection would be too high. The number of free reactive groups like amine or carboxyl groups available on the polymer mainly determines the upper limit. For example, a preferred biopolymer, gelatin, can accommodate a load of about 5 flavonoid molecules per 100 amino acids. Gelatin can be modified to increase the number of available free reactive groups. Too high loads may result in a too high hydrophobicity of the biopolymer reducing its water-solubility.

Preferably the load is about between 1 and 7.5 flavonoid molecules per 100 amino acids.

Gelatins which can be used as the water-soluble biopolymer are acid or lime treated skin or bone gelatins from mammals or cold-blooded animals. Such gelatins are common in the prior art and are well described in literature.

Also many chemically modified gelatins are known from the prior art like for example trimellitated, succinylated, acylated, alkylated, phthalated gelatin. Modified gelatins can advantageously be used as long as they do not elicit detrimental reactions like immune reactions when applied to the skin. Succinylated gelatins for example are applied as blood plasma expanders, and are medically and immunologically acceptable gelatin.

Modified gelatins in which the amount of free amine-groups has been increased can be used to increase the load of flavonoid compound on the gelatin. EP 0,487,686 describes a method to convert carboxyl groups into amines. Spacers can be used to increase the amount of free reactive groups of a biopolymer like gelatin. A spacer is a molecule which can be covalently linked to reactive groups of the biopolymer, like carboxyl groups or amine groups of gelatins, said spacer molecule containing at least two reactive groups like amine-groups or carboxyl groups, thus increasing the amount of available active groups. Spacer molecules are exemplified by amino acids like lysine, glutamic acid or aspartic acid, but are not limited to such structures. For example, activated esters of N-hydroxy-succinimide (NHS) can be used to form peptide linkages between functional groups, like carboxyl groups and primary amines, as is well known (Anderson et al., 1964, *J. Am. Chem. Soc.* **86**:1839-1842, US 5,366,958). NHS can be activated by forming an ester bond between NHS and the functional group (e.g. dihydroxybenzoic acid or an analogue thereof) which should be superimposed onto those of gelatin. Coupling of an activated NHS ester to amino groups of gelatin yields a chemically modified gelatin while N-hydroxy- succinimide is liberated. As another example, the functional group (e.g. flavonoid) can be coupled to lysine, and the carboxyl group of the modified lysine can then be activated with NHS and coupled to gelatin; alternatively, lysine can be bound to gelatin as a branch, and its two amino groups can be used to bind flavonoid molecules.

Other biopolymers with free amine groups, like casein, sericin, soluble collagen, and the like, can be used, although there is a preference for gelatin or collagen because of their low antigenicity and immunogenicity. If there is a desire to avoid immune reactions even further, for example in case of individuals suffering from (auto-)immune diseases, gelatins incapable of helix-formation can be advantageously used. This can be achieved by chemically modifying gelatins to impair helix formation or, preferably, by using gelatins produced by recombinant techniques as described in EP 1,063,665. Such recombinant gelatins may lack the presence of prolines in the peptide backbone or hydroxylation to hydroxy-proline can be reduced or prevented or avoided. Such recombinant gelatins are preferred over modified gelatins because they less likely to elicit immune reactions.

A further advantage of gelatins having no or little hydroxy-proline is that such gelatins have a low gelling temperature or do not gelate at all. Less or no gelation makes it possible to use a wider concentration range of the inventive flavonoid-gelatin compound in cosmetic applications. In that respect also gelatin derived from creatures living in cold water can be advantageously used since their gelatin has a naturally low hydroxy-proline content, and thus a low gelling temperature and immunogenicity or antigenicity.

The depth of penetration in the skin can be advantageously regulated by controlling the molecular weight of the gelatin preventing it from entering the bloodstream. Prior art applications of flavonoids do not include a method to control the skin penetration, but allow skin penetration freely. US 5,403,944 uses a polymeric UV absorber, but no flavonoid. Even shallow penetration of the skin is prevented.

High molecular weight of gelatins, over 200 kD, are less preferred. Such large molecules may give problems as to insufficient penetration in cosmetic applications. The molecular weight of conventional natural or modified gelatins can be regulated by for example hydrolysis or by enzymatic breakdown of the gelatin. The desired average molecular weight can be regulated satisfactorily, however the width of the molecular weight distribution can be problematic. After breakdown of the gelatin too small polypeptide (<= 5kD) or too big protein chains can be removed by known techniques like ultrafiltration, dialysis, noodle washing and the like. Recombinant produced gelatins have the additional advantage that the molecular weight and the distribution can be regulated precisely. Another method to regulate skin penetration is to adjust the hydrophobicity of the protein or polypeptide by chemical modification of said protein or polypeptide, covalently linking hydrophilic or hydrophobic groups to said protein or polypeptide.

The advantage of using flavonoids as the UV-absorbing component is that this class of chemicals comprises compounds that are capable of absorbing UV-A as well as those that are capable of absorbing UV-B radiation. In addition certain flavonoids are capable of chelating metal ions and/or scavenging radicals. Since the skin is an immunologically active organ, it is desirable to keep sunscreen formulation simple, avoiding exposure to many different compounds. Also the water solubility of the new compound contributes to this desired simplicity, since no oils or other organic solvents used in the prior art are necessary to dissolve the flavonoid.

In the prior art different definitions of the term 'flavonoids' are used. We use the term flavonoid to designate flavanes, flavanones and flavones and their derivatives.

Flavanes have the following characteristic basic structure:

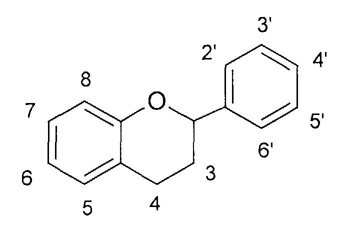

Flavanones have the following characteristic basic structure:

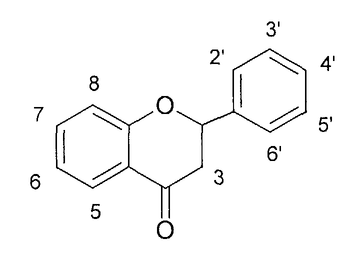

Flavones have the following basic structure:

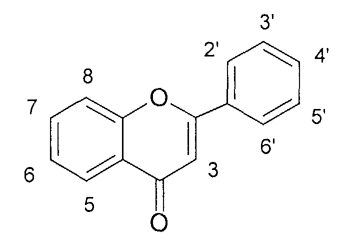

Structures can be chosen based on the desired functionality. As UV-A or UV-B absorbers flavonoid structures and derivatives can be selected from, but are not limited to, those described in EP 1,055,415 and WO 99/25316.

As metal-ion chelators any flavonoid comprising a catechol (3',4'-dihydroxyphenyl) group and optionally a 4-keto-5-hydroxy region can be selected. For example, flavonoids containing a catechol group include the flavones luteolin, fisetin, quercetin, robinetin, gossypetin and myricetin and the flavanones eriodictyol (2,3-dihydroluteolin) and taxifoline (2,3-dihydroquercetin). Flavonoids containing a 4-keto-5-hydroxy function include the flavones chrysin, apigenin, luteolin, campherol, quercetin, morin, gossypetin and myricetin and the flavanones eriodictyol and taxifoline. Most preferred flavonoid is the flavanol quercetin, since it is both a flavanol, and has the preferred catechol and 4-keto-5-hydroxy function and is abundantly present in nature. Flavanols are flavones that have a hydroxy group in the 3-position.

The inventive water-soluble polymer to which at least one flavonoid compound is linked can be advantageously applied in cosmetic preparations to protect the skin or hair from UV radiation.

Various cosmetic preparations for skin protection comprising UV-absorbing compounds are available in the market as lotions, emulsions, creams, milks, gels and the like. These may contain oil and/or alcohol. Also aerosols or sticks are known to be used. All such cosmetic preparations may function as a medium to apply the inventive flavonoid biopolymer. As explained before, applying the inventive flavonoid biopolymer makes the use of oils or alcohols obsolete, and even helps to prevent unwanted effects like immune reactions to such substances or reduced barrier functions of the skin.

The inventive flavonoid biopolymer is therefore preferably applied as, but not limited to, oil-less preparations like hydrogels.

The cosmetic preparation comprising the inventive flavonoid biopolymer can contain in addition to said biopolymer a second, conventional, UV-absorbing compound. This can be a UV-A, UV-B or broadband UV absorbing compound as described in for example EP 1,055,412. Other additives as applied in the art can advantageously be used in combination with the flavonoid biopolymer of the invention.

It will be clear to a person skilled in the art that the advantage of linking compounds to a water-soluble biopolymer like gelatin is not limited to UV-absorbing agents. For example, vitamins like vitamin C and vitamin E are normally added in cosmetic preparations as anti-oxidants. Although they do not impose a danger to the human organism, their function is reduced when such vitamins are allowed to diffuse away through the skin. Such compounds can also be advantageously linked to for example gelatin.

To our surprise quercetin could be successfully linked to gelatin using the Mannich reaction, condensating formaldehyde or another aldehyde such as C₁-C₆ alkanals in the presence of gelatin and quercetin at a temperature between 30°C and 80°C, thus linking said quercetin to the primary amine groups of gelatin. It is well known to the skilled organic chemist that addition can occur to each of the positions ortho or para to a hydroxy group. It was expected that subsequently a second amine group of gelatin would be linked to the quercetin molecule resulting in a crosslink. Surprisingly such crosslinking did not occur.

Preferably at least 1.5 equivalent up to five equivalents of aldehyde are used per equivalent of flavonoid, in particular between 2 and 4 mole equivalents.

It was known already that primary and secondary amines can be added to phenols or polyphenols using the Mannich reaction, in which said amine and (poly)phenol are heated in the presence of an aldehyde. GB 1,110,387 and GB 1,066,297 describe therapeutically useful derivatives of rutin obtained by the Mannich reaction using formaldehyde and a primary or secondary amine. It is stated that addition occurs preferably at the 5', 2' and 8 positions. DE 197 55 035 discloses a therapeutically useful water-soluble 3-methylquercetin derivative obtained by the Mannich reaction using formaldehyde and secondary amines such as morpholine and piperidine. Addition is stated to occur preferably at the 8 site, but also at 6, 2' or 5'. However, linking flavonoid compounds to biopolymers containing primary amino groups applying a Mannich reaction was not disclosed or suggested by the prior art.

NMR-studies showed that in the process of the invention, the Mannich reaction addition occurred only at the 8-position. This is not expected since normally some addition would occur also at the less preferred positions.

Mannich reactions are preferably carried out with secondary amines. Primary amines can undergo the Mannich reaction twice, first reacting to form a secondary amine which can then react further. Unexpectedly, this second step did not take place in the synthesis.

The addition of flavonoids to gelatin with the Mannich reaction can be performed at temperatures between 30°C and 80°C. A reaction temperature of 70°C is preferred. A too long reaction time or a too high temperature results in undesired red-colored by-products. Excessive reaction times can be prevented by the use of an organic co-solvent like an alcohol, an ether or a cyclic ether or ethyl acetate in water/co-solvent ratio between about 6/1 and 2/1. Too high ratios will reduce the reaction speed too much while at lower ratios gelatin will precipitate. Preferably ethanol is used. Preferably, the polypeptide contains an average of at least one lysine residues per 100 amino acid residues of the polypeptide.

The use of a synthetic route comprising the addition of an aldehyde like formaldehyde would not be an obvious choice of a person skilled in the art. For example formaldehyde and glutaraldehyde are well known crosslinkers of gelatin. Formaldehyde is used in photographic developers to 'harden' the gelatinous photographic layers, that is to crosslink the gelatin rendering it insoluble.

Crosslinking of gelatin by the aldehyde was effectively prevented according to the invention by performing the synthesis at neutral or acidic pH between about 1 and about 7, preferably between 3 and 6.5, most preferably between 5 and 6.

Another problem is that an excess of aldehyde of 2-4 times the molar amount of flavonoid has to be added for the reaction to proceed in a reproducible manner. Too large excess of the aldehyde will result in immediate crosslinking of gelatin. Depending on the composition of the starting solution (concentration gelatin from about 1 to 10%, water/co-solvent ratio between about 6/1 an 2/1, pH from about 1-7) the maximum amount of aldehyde which can be added without crosslinking the gelatin will vary. For example, for a 5% gelatin solution with a ethanol-water ratio of 1/3 and a pH of 5.5 a maximum of about 30 millimolar formaldehyde can be added.

Also too high starting concentrations of quercetin and aldehyde will result in crosslinking. For example, a starting solution (pH of 5.5) of 5% gelatin with a ethanol-water ratio of 1/3, 15 millimolar quercetin and 15 millimolar formaldehyde resulted in immediate crosslinking of the gelatin.

Although preventing immediate crosslinking as described above, drying the flavonoid-gelatin compound still results in crosslinking by said excess aldehyde. This could be avoided by adding NaHSO₃ before purifying the flavonoid-gelatin product with an anion exchange resin. The ion exchange resin binds the NaHSO₃-aldehyde adduct effectively removing the aldehyde from the flavonoid-gelatin product. To avoid crosslinking of the gelatin on the column, the anion exchange resin should not be used in the OH-form, but can be used in the Cl-form. In principle any anion exchange column can be used provided it sufficiently retains the unreacted quercetin and the NaHSO₃-aldehyde adduct and has no adverse effect on the quercetin-gelatin compound, like crosslinking on the column.

Thus solving all these problems we were able, against expectations, to synthesize and purify the UV absorbing water-soluble biopolymer of the invention.

### Example 1: Manufacturing a quercetin-gelatin compound with a load of 0.1 mmol quercetin per gram gelatin

10 grams of a lime bone gelatin was dissolved at 60°C in 150 ml of demineralized water. 50 Milliliter of ethanol was added to the solution.

To the clear solution (pH 5.5) 338 mg of quercetin bis-hydrate was added, forming a suspension. Then, slowly, 243 microliter of a formaldehyde solution (37% in water) was added while stirring. The suspension was heated to 70°C. After 15 to 45 minutes the precipitated quercetin disappeared and the mixture turned clear yellow. After 90 minutes, 416 milligram of NaHSO₃ was added and the mixture was stirred for an additional hour.

Then the yellow solution was poured onto an ion exchange resin Amberlite IRA-93 (Cl-form) which was previously eluted with 60°C warm demineralized water. The reaction mixture was eluted through the resin with demineralized water. The resin was rinsed with one column-volume of demineralized water.

The eluted solution was dried overnight at 55°C, and a quercetin-gelatin compound with a load of 0.1 millimol quercetin per gram gelatin, or 1 molecule quercetin per 100 amino acids, was obtained with a yield of 100%.

NMR analysis surprisingly showed that quercetin was linked to amine groups of gelatin only at the 8-position of quercetin.

### Example 2: Synthesis of a rutin-gelatin compound with a load of 0.1 millimol rutin per gram gelatin, or 1 molecule rutin per 100 amino acids.

Synthesis was performed as in example 1, except that rutin was added in the same molar amount as quercetin. A rutin-gelatin compound with a load of 0.1 millimol rutin per gram gelatin, or 1 molecule rutin per 100 amino acids, was obtained with a yield of 100%.

### Example 3: Synthesis of a quercetin-gelatin compound with a load of 0.2 millimol quercetin per gram gelatin, or 2 molecules quercetin per 100 amino acids.

Synthesis was performed as in example 1, except that when the clear yellow solution was formed after addition of quercetin, an addition amount of 338 mg quercetin was added, resulting in a suspension. This was again allowed to react until the solution was a clear yellow, after which the synthesis was proceeded as in example 1.

A quercetin-gelatin compound with a load of 0.2 millimol quercetin per gram gelatin, or 2 molecules quercetin per 100 amino acids, was obtained with a yield of 100%.

### Example 4: Synthesis of a quercetin-taxifolin-gelatin compound with a total load of 0.2 millimol flavonoid per gram gelatin.

Synthesis was performed as in example 1, except that when the clear yellow solution was formed after addition of quercetin, an addition amount of 1 millimol taxifolin (2,3-dihydroquercetin) was added, resulting in a suspension. This was again allowed to react until the solution was a clear yellow, after which the synthesis was proceeded as in example 1.

A quercetin- taxifolin-gelatin compound with a load of 0.1 millimol quercetin and 0.1 millimol taxifolin per gram gelatin was obtained with a yield of 100%.

### Example 5: UV-A protection by a quercetin-gelatin compound with a load of 0.1 millimol quercetin per gram gelatin.

The test is performed on volunteer subjects with normal skin types III (Burns moderately, tans gradually) or IV (Burns minimally; tans well). The subjects have no history of photosensitivity, are not using systematic medications and have not been exposed to direct sunlight for the previous 6 weeks.

Two test sites of 5x10 cm, each test site divided in 6 sub-sites, are marked on the back of the subjects between the waist line and the shoulder blades. One test site is not treated and therefor unprotected. On the other test site 100 mg sunscreen product is applied uniformly to the skin so that the amount of the UV-absorbing compound applied is about 2 mg per m2. In case of the quercetin-gelatin compound the amount applied is such that also in this case 2 mg/m2 of the linked quercetin is applied, The applied sunscreen is allowed to dry for about 15 minutes.

A series of six doses of UV-A radiation, increasing in energy is applied to each test site using a UV-A Sellas Sun 2000 (irradiance about 50 milli Watt/cm² at 30 cm). Doses applied are from 33 to 8 joule/cm2, randomly applied over the 6 sub sites, each exposure being 25% less than the previous.

The MPD (minimal pigmenting dose) of each test site is determined visually after 3 hours of exposure. The MPD is defined as the quantity of radiation energy required to produce the first unambiguous pigmented reaction.

The SPF (Sun protection factor) is calculated as the quotient of the MPD of the unprotected skin and the MPD of the protected skin.

The results are summarized in the following table:

| | MPD (Joule/cm²) | | SPF |
|---|---|---|---|
| | protected | unprotected | |
| Group 1: Quercetin | | | |
| Volunteer A | 14 | 8 | 1.8 |
| Volunteer B | 19 | 8 | 2.4 |

| Group 2: Quercetin-gelatin compound | | | |
|---|---|---|---|
| Volunteer C | 33 | 10 | 3.3 |
| Volunteer D | 25 | 8 | 3.1 |

The results show that the quercetin-gelatin compound of the invention provides good protection from UV-A radiation. It performed better than the free quercetin because of the higher stability under UV exposure of the quercetin linked to gelatin.

### Example 6: Penetration depth of quercetin-gelatin into the skin

### A/ color reaction

The essential condition is the existence of specific detection method, which can be used to visualize the presence of particular sunscreen component. In case of quercetin one can take advantage of the existence of ortho-dihydroxybenzene (catechol) configuration on the benzene ring B. Different colorimetric methods for catechols have been described. We choose the reaction described in J. Invest Dermatol., 70:197, 1978, (S. Pavel et al.).

### B/skin penetration

Skin penetration study were executed using the following preparations:
1. Quercetin (1%) in cremor cetomacrogolis FNA (CMC)
2. Quercetin-gelatin (small molecular weight fraction) (10-20%) in CMC.
3. Quercetin-gelatin (large molecular weight fraction) (10-20%) in CMC.
4. Catechol (1%) (as a penetration marker) in CMC.

The preparations were applied to the skin (buttocks) of three volunteers. The penetration times were 30 min, 1 hour and 3 hours. After each penetration time a biopsy (4 mm) was taken after local anesthesia. The biopsy material was frozen in liquid nitrogen and kept for the further analysis.

### Microscopy:

The biopsy material were cut in consecutive slices of 8 um thick. The slices were incubated in a selected solution to achieve the color characteristic of catecholic structures. The depth of penetration was measured visually and expressed as a percentage penetrated epidermis, as follows:

| Penetration depth | 30 min | 1 hour | 3 hour |
|---|---|---|---|
| 1. Quercetin (1%) | 30 % | 50 % | 80% |
| 2. Quercetin-gelatin (small MW) | 10 % | 30 % | 50 % |
| 3. Quercetin-gelatin (large MW) | 5 % | 20 % | 30 % |
| 4. Catechol (1%) (penetration marker) | 40 % | 60 % | 90 % |

### Example 7: Chelating capacity of quercetin-gelatin

The amount of free Fe²⁺ and Fe³⁺ can be determined by ion-chromatography.

Gelatin has a very high affinity to Dionex column material that is used in the determination of iron ions. Gelatin will not elute from the column and thereby changes the characteristics of the column so that the separation is not stable. To prevent this a solid state extraction (SPE) column is used to separate the metal ions from the gelatin matrix. After this pretreatment the sample is conservated with 10 millimolar hydrochloric acid. The sample is then injected into the ion-chromatograph. The metal-ions form a complex with the pyridine-2,6-dicarboxylic acid (PDCA). The complexed iron ions are then separated by their differences in affinity to the column material, which has as well anion as cation characteristics (mixed mode). After the separation 4-(2-pyridylazo)resorcinol (PAR) is added that absorbs light in the visual range. The signals obtained at the retention times known for Fe²⁺ and Fe³⁺ are proportional to the concentrations, which are calculated from a calibration curve.

### Sample pretreatment:

A Dionex OnGuard-RP P/N 039595 column was conditioned with 5 ml methanol and 10 ml de-ionized water. 9 ml sample is brought onto the column. The first 3 ml eluate is discarded, the next 6 ml is collected. 5 ml of the eluate is quantitatively pipetted into a 10 ml volumetric flask and 20 mM hydrochloric acid was added up to 10.00 ml.

### Determination of the amount of free Fe2+ and Fe3+ by ion chromatography (derived from Dionex technical note nr. 10):

Equipment used is a Dionex DX500 system comprising a GP40 gradient pump, an AS40 Autosampler, an LC30 column oven, an AD20 spectrophotometric detector, an ED40 electrochemical detector and a PC 10 pneumatic post-column reagent pump.

Eluent is prepared by 5 times dilution of Dionex MetPac Eluent concentrate P/N 46008.

Post-column reagent is prepared by dissolving 0.12 gram PAR in 1.0 liter MetPac Post-column Diluent P/N 46094. A clear solution is obtained by ultrasonic treatment.

The ion-exchange column that is used is a Dionex IonPac CS5A analytical column P/N 46100 with a P/N 46104 pre-column. The post-column reactor is a 375 microliter knitted reaction coil (Dionex).

50 microliter of the pretreated sample is injected. The eluent flow is 1.2 milliliter per minute and the post-column reagent flow is 0.7 milliliter per minute. Detection is done at a wavelength of 530 nm.

All solutions were purged with helium during preparation and kept under nitrogen atmosphere to prevent oxidation. The following test solutions were prepared in ethanol:water, ratio of 1:3.
- Solution 1: 1 millimol FeSO₄ per liter
- Solution 2: 10 gram lime bone gelatin per liter
- Solution 3: 10 gram gelatin-quercetin compound of example per liter
- Solution 4: 1 millimol quercetin per liter
- Test sample 1a: Solution 1 : ethanol-water solution (1:3) = 1:1
- Test sample 2a: Solution 1 : solution 2 = 1:1
- Test sample 3a: Solution 1 : solution 3 = 1:1
- Test sample 4a: Solution 1 : solution 4 = 1:1

In the same way, test samples 1b, 2b, 3b and 4b were prepared with Fe₂(SO₄)₃ instead of FeSO₄. The test samples were mixed for 10 minutes at 37°C. The amounts of free Fe2+ or Fe3+ were determined by the method described above. The results are summarized in the following table:

| | Amount free Fe2+ (millimol per liter) | Amount free Fe3+ (millimol per liter) |
|---|---|---|
| Test samples 1a,b | 0.50 | 0.50 |
| Test samples 2a,b | 0.45 | 0.42 |
| Test samples 3a,b | 0.12 | 0.15 |
| Test samples 4a,b | 0.16 | 0.20 |

The results show that the chelating capacity of quercetin is still present after binding to gelatin. Free iron can also be determined by NMR, with the same result as described above.

## Claims

1. A UV radiation absorbing compound wherein at least one flavonoid is covalently linked to a polypeptide.

2. A compound according to claim 1, wherein said flavonoid is linked through an alkylidene group attached to a flavonoid and to a polypeptide amino function

3. A compound according to claim 1 or 2, wherein said polypeptide comprises between 20 and 2,000 amino acid residues.

4. A compound according to claim 3, wherein said polypeptide comprises between 30 and 1,000 amino acid residues.

5. A compound according to any one of claims 1-3, wherein said polypeptide comprises at least one lysine residue.

6. A compound according to any one of claims 1-4, wherein at least one flavonoid contains a 4-keto-5-hydroxy function and/or a catechol function.

7. A compound according to any one of claims 1-5, wherein said flavonoid comprises a flavone and a flavanone.

8. A compound according to claim 7, wherein said flavone is quercetin.

9. A compound according to any one of claims 1-7, wherein at least one hydroxy group of said flavonoid is chemically modified, said modification comprising alkylation, hydroxyalkylation, acylation, glycosylation, or a combination thereof.

10. A compound according claim 9, wherein said flavonoid is rutin.

11. A compound according to any one of claims 1-10, wherein the amount of flavonoid linked to said polypeptide is from 0.15 to 15 flavonoid molecules per 100 amino acids, preferably from 1 to 7.5 flavonoid molecules per 100 amino acids.

12. A compound according to any one of claims 1-11, wherein the polypeptide is selected from casein, sericin, soluble collagen and gelatin.

13. A compound according to claim 12, wherein said polypeptide is collagen or gelatin which is free from helical structure.

14. A compound according to any one of the preceding claims, wherein an anti-oxidant, such as vitamin C or vitamin E, is covalently linked to said polypeptide.

15. A cosmetic composition for protection against UV radiation comprising a cosmetically and dermatologically acceptable carrier containing a compound according to any one of the preceding claims.

16. A process for preparing a UV radiation absorbing compound according to any one of claims 1-14, comprising reacting a flavonoid with a polypeptide in the presence of an aldehyde.

17. A process according to claim 16, wherein the reaction is performed at a pH between 1 and 7, preferably between 5 and 6.

18. A process according to claim 16 or 17, wherein the molar amount of aldehyde added is between 1.5 and 4 times the molar amount of the flavonoid.

19. Use of a compound according to anyone of claims 1-14, for preventing the detrimental effects of UV radiation.
